**Europäisches Patentamt**

**European Patent Office**

**Office européen des brevets**

(11) Numéro de publication : **0 012 637**
**B1**

(12) **FASCICULE DE BREVET EUROPÉEN**

(45) Date de publication du fascicule du brevet :
**25.07.84**

(21) Numéro de dépôt : **79400841.7**

(22) Date de dépôt : **09.11.79**

(51) Int. Cl.³ : **A 61 B 10/00**

(54) **Cellule de migration cellulaire.**

(30) Priorité : **06.12.78 FR 7834345**

(43) Date de publication de la demande :
**25.06.80 Bulletin 80/13**

(45) Mention de la délivrance du brevet :
**25.07.84 Bulletin 84/30**

(84) Etats contractants désignés :
**AT BE CH DE GB IT LU NL SE**

(56) Documents cités :
**DE-A- 2 624 627**
**FR-A-   224 008**
**US-A- 3 066 672**

(73) Titulaire : **Laurent, Philippe**
**92, Grande-rue**
**F-69000 Oullins (FR)**

**INSTITUT PASTEUR DE LYON**
**77, rue Pasteur**
**F-69000 Lyon (FR)**

(72) Inventeur : **Laurent, Philippe, Dr.**
**92, Grande-rue**
**F-69000 Oullins (FR)**

(74) Mandataire : **Corre, Jacques Denis Paul et al**
**Cabinet Regimbeau 26, Avenue Kléber**
**F-75116 Paris (FR)**

## Description

La présente invention concerne une cellule de migration cellulaire, en particulier destinée à l'étude de la migration leucocytaire et monocytaire.

La cellule selon l'invention trouve notamment son application dans le domaine de l'expertise clinique et biologique en pharmacotoxicologie humaine et/ou animale, dans les bilans d'études des fonctions leucocytaires en clinique infectieuse et allergologique, ainsi que dans le domaine de la recherche fondamentale sur les fonctions des leucocytes.

Dans la technique antérieure relative à un tel type de matériel, on connaît la chambre de migration de H. Senn (Rev. Fr. Et. Clin. Biol. 1969, 14, 373-377) et la chambre de H. Dunsky « Skin Chamber » (J. Allergy Clin. Immunol. 1978, 26, 2 127-130). Ces cellules de la technique antérieure sont essentiellement constituées par une chambre unique sans voies d'entrée et de sortie pour la chambre Senn, et sont directement appliquées sur une abrasion cutanée « skin window », sans interposition d'un quelconque matériau pour la chambre Dunsky.

Il convient par ailleurs de noter qu'un grand nombre de techniques d'études de la migration cellulaire à partir d'une abrasion cutanée « skin window » ont été décrites depuis la publication initiale de Rebuk J. W. (Ann. N. Y. Aca. Sci., 1955, 59, 757-794).

Le principe de base de l'ensemble de cette technique, dite directe, comporte la seule application d'une lamelle de verre directement sur l'abrasion cutanée, puis consiste à numéroter les éléments cellulaires adhérant à cette lamelle de verre, après coloration adéquate.

En outre, la demande de brevet allemand n° 26 24 627 décrit une cellule de prélèvement cellulaire comprenant une chambre munie à sa base d'une ouverture plane, d'un conduit d'entrée et d'un conduit de sortie reliés entre eux par un circuit de connexion extérieur incorporant une pompe. Le but de l'invention est d'améliorer un tel type de cellule en vue notamment de permettre une thermo-régulation du milieu réactif liquide, une bonne homogénéisation de ce dernier au cours de son agitation, d'assurer un meilleur renouvellement des facteurs actifs sur la mobilisation leucocytaire et de pouvoir éviter la pénétration des leucocytes dans la chambre interne et donc leur mise en suspension dans le milieu réactif actif.

Conformément à la présente invention, la cellule de migration cellulaire, destinée en particulier à l'étude de la migration leucocytaire et monocytaire, du type comportant au moins une chambre de migration qui comprend une base munie d'une ouverture à bord plan, un premier conduit débouchant dans chaque chambre au voisinage du sommet de celle-ci et un second conduit débouchant aussi dans chaque chambre sur la paroi latérale de celle-ci sensiblement à mi-hauteur entre son sommet et sa base, lesdits conduits étant reliés à un circuit de connexion extérieur incorporant une pompe de circulation, est caractérisée en ce qu'elle contient en outre une pluralité de conduits débouchant dans chaque chambre au voisinage immédiat de sa base et une chambre externe enveloppant l'ensemble de la ou des chambre(s) de migration à l'exception de l'ouverture de base, les chambres externe et de migration(s) étant réunies de façon étanche, en particulier au niveau de leur base, et délimitant entre elles un espace destiné à être traversé par un fluide régulateur de température circulant entre un conduit d'entrée et un conduit de sortie de la chambre externe, en ce que l'ensemble des conduits débouchant au voisinage de la base de chaque chambre de migration est connecté au second conduit débouchant à mi-hauteur de chaque chambre de façon à assurer une agitation permanente par flux liquidien du milieu réactif liquide contenu dans chaque chambre de migration et en ce que le bord de l'ouverture ménagée dans la base de chaque chambre de migration est réalisée de manière à autoriser soit la mise en place de ladite cellule sur la peau avec interposition d'un filtre micropore de porosité de l'ordre de 3 à 5 µm, soit éventuellement la mise en place directe de la cellule sur une muqueuse.

D'autres caractéristiques et avantages de la cellule selon l'invention apparaîtront à la lecture de la description détaillée faite ci-après en référence aux dessins annexés, et sur lesquels :

la figure 1 représente une vue en perspective d'un mode de réalisation particulier de la cellule selon l'invention ;

les figures 2 et 3 représentent des coupes de cellules du type de celle représentée à la figure 1 ;

la figure 4 est une vue de dessous de la cellule selon l'invention, et

la figure 5 illustre la mise en place d'une cellule selon l'invention sur le bras d'un témoin.

La cellule de migration cellulaire 10 selon l'invention contient au moins une chambre interne 12. Sur les dessins annexés, la cellule 10 ne comporte qu'une seule chambre intérieure 12, mais il est parfaitement clair que la présente invention s'étend également à des cellules de migration cellulaire incorporant plusieurs chambres internes du type de la chambre 12 décrite ici. Cette chambre interne 12 présente à sa base une ouverture 14 et comporte en outre un premier conduit d'entrée 16 débouchant au voisinage du sommet de ladite chambre 12, ainsi qu'un second conduit d'entrée 18 débouchant sur la paroi latérale de la chambre interne 12, sensiblement à mi-hauteur entre le sommet et la base de cette dernière. La chambre interne 12 comporte enfin une pluralité de conduits de sortie 20 débouchant au voisinage immédiat de la base de ladite chambre 12.

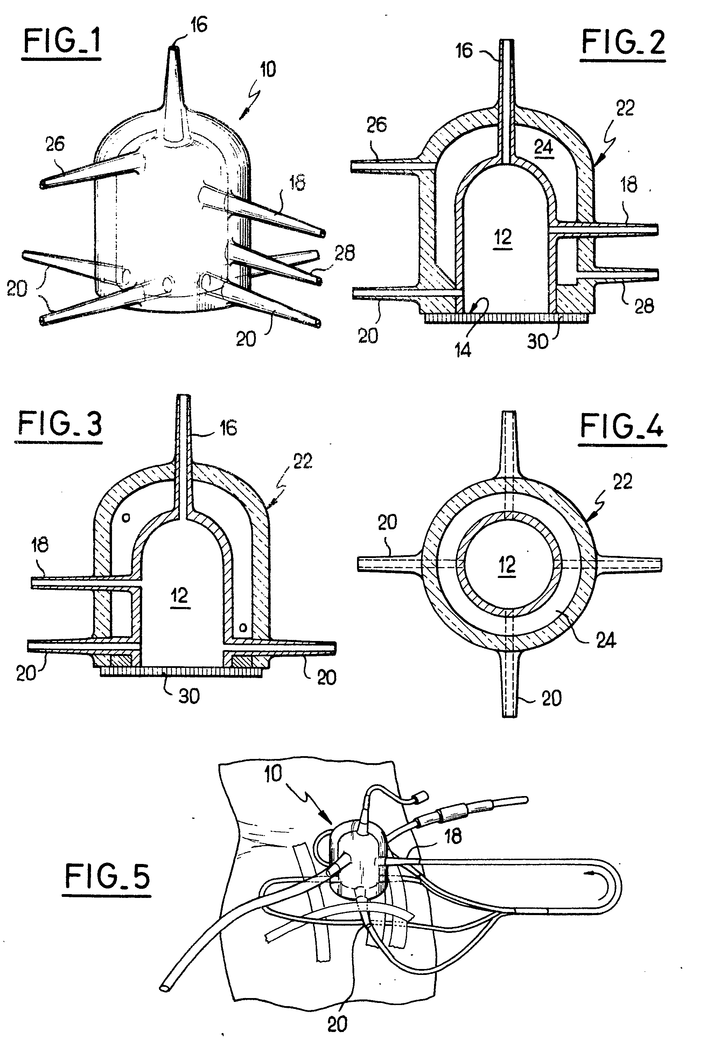

La cellule 10 comporte également une chambre externe unique 22 enveloppant l'ensemble de la ou des chambres internes 12. Comme cela apparaît sur les figures 2 et 3, les chambres externe 22 et

2

interne(s) 12 sont réunies de façon étanche afin de définir entre elles un espace 24 destiné à être traversé par un fluide de régulation de température. L'espace 24 ménagé entre les parois internes de la chambre externe 22 et les parois externes de la chambre interne 12 doit être suffisant pour permettre une bonne circulation du liquide de régulation de température entre un conduit d'entrée 26 et un conduit de sortie 28 de la chambre externe 22. Cette caractéristique est importante, étant donné que le volume interne de la ou des chambre(s) interne(s) doit être constant au cours de l'utilisation de la cellule et ne doit donc pas être sensible aux variations de volume liées à des différences de température. Cette régulation de température de la cellule de migration selon l'invention permet également de maintenir une température optimale pour le déroulement des réactions enzymatiques liées au réactif déposé dans la chambre interne. Il est donc ainsi possible, en faisant varier la température du liquide de régulation, et partant de la cellule, d'accélérer et/ou de bloquer telle ou telle réaction enzymatique.

Selon un mode de réalisation avantageux, les conduits d'entrée et de sortie de la chambre interne traversent l'espace dans lequel circule le fluide de régulation de température.

Selon une autre caractéristique de la présente invention, l'ensemble des conduits de sortie 20 de la chambre interne 12 est connecté au second conduit d'entrée 18 de façon à assurer une agitation permanente, par flux liquidien, du milieu réactif liquide contenu dans ladite chambre interne 12. Pour entretenir ce flux de liquide, le circuit de connexion comporte une pompe, non représentée, de type classique par exemple une pompe par étirement ou une pompe péristaltique.

Comme cela apparaît aux figures 2 et 3, la réunion entre chambre externe et chambre(s) interne(s), au niveau de leur base, est réalisée de manière à définir une surface plane. Cette surface plane est destinée à recevoir, comme illustré aux figures 2 et 3, un filtre micropore 30. Un tel filtre 30 peut être du type « micropore » présentant une porosité comprise entre environ 3 et environ 5 microns. Bien entendu, la porosité particulière sera choisie en fonction du type des cellules étudiées. Le filtre micropose 30 est fixé de façon étanche sur la surface plane de réunion des bases des chambres externe 22 et interne(s) 12, par exemple par collage. Un tel filtre permet de recueillir les cellules qui ont migré à partir de l'abrasion cutanée. On peut ainsi compter à l'intérieur du filtre les cellules déplacées et déterminer un index de migration cellulaire. Ce filtre permet en effet de recueillir les cellules migrantes (polynucléaires neutrophiles, monocytes, basophiles, etc.) présentes dans le tissu sous-jacent à l'abrasion cutanée. Les cellules ont, en effet, la possibilité de s'infiltrer à l'intérieur des pores au filtre 30, cette migration de cellules dans les pores étant proportionnelle à la concentration de substances attractantes produites dans le réactif sus-jacent au filtre selon le modèle enzymatique. En fin d'expérience, le filtre 30 est prélevé sur la cellule 10, puis on procède à une numération des éléments cellulaires après transparisation du filtre et coloration. La numération des éléments cellulaires est rapportée à la distance de la migration.

Selon un mode de réalisation particulièrement avantageux de la cellule selon l'invention, les conduits de sortie 20 de la chambre interne 12 sont réalisés sous la forme de tubes capillaires de diamètre sensiblement identique. Dans pareil cas, le second conduit d'entrée 18 de la chambre interne 12 est également réalisé de façon avantageuse sous la forme d'un tube capillaire présentant alors un diamètre sensiblement quatre fois égal au diamètre desdits tubes capillaires de sortie 20 de la chambre interne 12. La nature capillaire des sorties 20 et de l'entrée 18 permet d'obtenir une agitation relativement faible du milieu liquide réactif de la cellule.

Les conduits de sortie 20 doivent être disposés le plus près possible de la surface supérieure du filtre 30 afin de réaliser un flux liquidien tangentiel à la surface de ce filtre. Ceci permet en effet de réaliser un balayage de la partie supérieure du filtre et de lutter contre la force de pesanteur qui s'applique sur le filtre, en déplaçant le point d'application de cette force tangentiellement au filtre.

Le second conduit d'entrée 18 de la chambre interne 12 situé sur le flanc de la cellule 10 doit se situer sensiblement à mi-hauteur afin de maintenir un certain niveau de réactif liquide à l'intérieur de la chambre interne 12.

Selon une autre caractéristique de la présente invention, le premier conduit d'entrée 16 de la chambre interne 12

Selon une autre caractéristique de la présente invention, le premier conduit d'entrée 16 de la chambre interne 12 présente un diamètre suffisamment large pour,permettre l'introduction dans la chambre interne 12 d'organes de contrôle de la température, de la pression et/ou du degré de saturation en oxygène, ainsi que des organes destinés à permettre des prélèvements de réactif. Il est donc ainsi possible de prélever à tout moment du réactif contenu dans la chambre interne 12, afin de procéder à tout dosage nécessaire. Il peut par exemple s'avérer intéressant de procéder au dosage des composants du système complément dosage de l'histamine libérée au cours d'une dégranulation des basophiles vis-à-vis d'un allergène. Dans un mode de réalisation particulier, le premier conduit d'entrée 16 de la chambre interne est équipé d'un organe de régulation du système des pressions à l'intérieur de ladite chambre. Grâce à la création d'un flux en circuit fermé du réactif liquide, il est possible par un simple ajustage du débit, de réguler la pression présente dans la chambre interne 12. Dans ce cas, le premier conduit d'entrée 16 de la chambre interne 12 peut être équipé d'une simple seringue contenant le réactif de la cellule de migration.

Dans un mode de réalisation avantageux de la présente invention, les conduits de sortie 20 de la chambre interne s'étendent radialement dans un plan parallèle à la surface plane de réunion des bases des chambres externes 22 et interne 12, avec un écartement angulaire sensiblement constant. Cette disposition particulière en étoile permet, comme illustré à la figure 5, une mise en place facile de la cellule par exemple sur le bras d'un témoin.

Selon un mode de réalisation particulièrement avantageux de la présente invention, les parois internes de la ou des chambre(s) interne(s) 12 sont traitées afin d'éviter l'adhésion des cellules telles que les polynucléaires neutrophiles ou les monocytes, sur lesdites parois, ce qui serait évidemment préjudiciable au comptage des cellules dans le milieu réactif. Dans la pratique, un traitement au silicone des parois internes conduit à des résultats tout à fait satisfaisants.

La cellule de migration selon la présente invention peut être déposée en périphérie sur la peau d'organismes humains ou animaux ou à l'intérieur de ces organismes, par exemple sur des muqueuses. Dans le cas de l'application de la cellule de migration sur des muqueuses, l'utilisation du filtre 30 interposé entre la chambre interne 12 et la muqueuse peut être supprimée, la muqueuse remplaçant en quelque sorte alors le filtre 30.

Le réactif destiné à être introduit dans la chambre interne 12 de la cellule selon l'invention, a pour base, quelle que soit l'application pratique de la cellule, un milieu de survie cellulaire tamponné et enrichi en éléments indispensables à la vie des cellules. Ce milieu de survie cellulaire peut être associé à un réactif proprement dit qui peut être choisi parmi :

— le sérum du patient ou de l'animal testé ;

— l'association de ce sérum et d'un activateur du système complément sérique producteur de facteurs cyto-attractants ;

— toute substance chimique ou molécule dont on veut étudier l'activité sur la migration cellulaire.

## Exemple d'application

### Etude du chimiotactisme des polynucléaires neutrophiles humains :

Cet examen a pour but de rechercher et d'analyser un déficit du chimiotactisme des polynucléaires neutrophiles au cours des états septiques. En préalable de cette étude, il convient d'étudier la migration des polynucléaires neutrophiles à partir d'une abrasion cutanée, réalisée par des moyens mécaniques, sur 20 témoins indemnes de toute affection médico-chirurgicale évolutive.

Le milieu réactif contenu dans la chambre interne 12 est composé :

— d'un milieu de survie cellulaire type Phelps à pH compris entre 7,09 et 7,11 ;

— du sérum du patient à tester à une dilution de 1/2, et

— d'un activateur du système complément : gammaglobulines humaines agrégées.

Le matériel utilisé dans cet examen est une cellule du type de celle, objet de la présente invention, équipée d'un filtre micropore ayant une porosité de 5 microns. Le temps de migration est de 3 heures et la température a été maintenue à 37 °C au cours de cette migration.

L'index chimiotactique se calcule selon la formule :

$$Ic = \text{nombre de cellules par couches} \times dn/\text{nombre de cellules sur chaque couche}$$

$dn$ = distance entre deux couches.

Les vingt témoins étudiés donnent une migration cellulaire moyenne de 13 à 37 microns (écart type 2-10) ce qui dans le présent système expérimental établit le 100 % de migration.

Le nombre moyen de cellules déplacées par cette technique est en moyenne de 841 par champ de lecture sur le filtre avec un écart type moyen de 387.

La distance moyenne de migration est de 58,39 microns avec un écart type moyen de 4,09.

Selon la même technologie, dix patients atteints de syndrome infectieux de gravités diverses ont été traités. Les résultats exprimés en pourcentage par rapport aux témoins sont :

6 patients index chimiotactique   33 à 35 %
2 patients index chimiotactique   20 à 24 %
1 patient  index chimiotactique   45 %
1 patient  index chimiotactique   80 %

Ceci en phase aiguë de leur agression microbienne.

Bien entendu, la présente invention ne se limite pas au mode de réalisation particulier et à l'application particulière décrits.

## Revendications

1. Cellule de migration cellulaire, destinée en particulier à l'étude de la migration leucocytaire et monocytaire, du type comportant au moins une chambre de migration (12) qui comprend une base munie

4

d'une ouverture (14) à bord plan, un premier conduit (16) débouchant dans chaque chambre (12) au voisinage de son sommet et un second conduit (18) débouchant aussi dans chaque chambre (12) sur la paroi latérale de celle-ci sensiblement à mi-hauteur entre son sommet et sa base, lesdits conduits (16, 18) étant reliés à un circuit de connexion extérieur incorporant une pompe de circulation, caractérisée en ce qu'elle contient en outre une pluralité de conduits (20) débouchant dans chaque chambre (12) au voisinage immédiat de sa base et une chambre externe (22) enveloppant l'ensemble de la ou des chambre(s) de migration (12) à l'exception de l'ouverture de base (14), les chambres externe (22) et de migration(s) (12) étant réunies de façon étanche, en particulier au niveau de leur base, et délimitant entre elles un espace (24) destiné à être traversé par un fluide régulateur de température circulant entre un conduit d'entrée (26) et un conduit de sortie (28) de la chambre externe (22), en ce que l'ensemble des conduits (20) débouchant au voisinage de la base de chaque chambre de migration (12) est connecté au second conduit (18) débouchant à mi-hauteur de chaque chambre (12) de façon à assurer une agitation permanente par flux liquidien du milieu réactif liquide contenu dans chaque chambre de migration (12) et en ce que le bord de l'ouverture (14) ménagée dans la base de chaque chambre de migration (12) est réalisée de manière à autoriser soit la mise en place de ladite cellule sur la peau avec interposition d'un filtre micropore (30) de porosité de l'ordre de 3 à 5 μm, soit éventuellement la mise en place directe de la cellule sur une muqueuse.

2. Cellule selon la revendication 1, caractérisée en ce que les conduits (20) débouchant près de la base de chaque chambre de migration (12) sont réalisés sous la forme de tubes capillaires de diamètre sensiblement identique.

3. Cellule selon l'une des revendications 1 et 2, caractérisée en ce que ledit filtre micropore (30) est fixé de façon étanche sur la surface plane de réunion des bases de chambres externe (22) et de migration(s) (12), par exemple par collage.

4. Cellule selon l'une des revendications 1 à 3, caractérisée en ce que les parois internes de chaque chambre de migration (12) sont traitées afin d'éviter l'adhésion des cellules, telles que les polynucléaires neutrophiles ou les monocytes, sur lesdites parois.

5. Cellule selon la revendication 4, caractérisée en ce que le traitement desdites parois est un traitement au silicone.

6. Cellule selon l'une des revendications 1 à 5, caractérisée en ce que les conduits (16, 18 et 20) de chaque chambre de migration (12) traversant l'espace (24) dans lequel circule le fluide régulateur de température.

7. Cellule selon l'une des revendications 1 à 6, caractérisée en ce que le premier conduit débouchant près du sommet de chaque chambre de migration (12) présente un diamètre suffisamment large pour permettre l'introduction dans chaque chambre de migration (12) d'organes de contrôle de la température, de la pression et/ou du degré de saturation en oxygène, et pour permettre des prélèvements de réactifs.

8. Cellule selon l'une des revendications 1 à 7, caractérisée en ce que le premier conduit débouchant près du sommet de chaque chambre de migration (12) est équipé d'un organe de régulation du système des pressions à l'intérieur de ladite chambre.

9. Cellule selon la revendication 8, caractérisée en ce que ledit organe de régulation du système des pressions est constitué par une seringue contenant le réactif de ladite cellule.

10. Cellule selon l'une des revendications 1 à 9, caractérisée en ce que les conduits débouchant près de la base de chaque chambre de migration (12) s'étendent radialement dans un plan parallèle à la surface plane de réunion des bases des chabres externe (22) et de migration(s) (12), avec un écartement angulaire sensiblement constant.

## Claims

1. Device for studying cellular migration, intended in particular for studying leucocytary and monocytary migration, of the type having at least one migration chamber (12) which comprises a base provided with a flat sided opening (14), a first duct (16) opening into each chamber (12) in the region of its top and a second duct (18) opening also into each chamber (12) on the lateral side wall thereof substantially at mid height between its top and its base, the said ducts (16, 18) being connected by an external connection circuit incorporating a circulation pump characterised in that it contains in addition a plurality of ducts (20) opening into each chamber (12) in the region close to its base and an external chamber (22) enclosing the assembly of the migration chamber or chambers (12) except at the base opening (14), the external chambers (22) and the migration chamber(s) (12) being connected in a pressure-tight manner, in particular at the level of their base, and delimiting between themselves a space (24) intended to be traversed by a temperature regulating fluid circulating between an inlet duct (26) and an outlet duct (28) of the external chamber (22), in that the assembly of ducts (20) opening in the region of the base of each migration chamber (12) is connected to the second duct (18) opening at mid height of each chamber (12) in a manner to ensure a permanent agitation by liquid flow of the reactive liquid medium contained in each migration chamber (12) and in that the edge of the opening (14) arranged in the base of each migration chamber (12) is provided in a manner to permit either the placing of the device on the skin with the interposition of a micro-porous filter (30) of a porosity in the order of 3 to 5 μm, or possibly positioning of the device directly on a mucous membrane.

2. Device according to claim 1, characterised in that the ducts (20) opening near the base of each migration chamber (12) are provided in the form of capillary tubes of substantially the same diameter.

3. A device according to either of claims 1 and 2, characterised in that the said micro-porous filter (30) is fixed in a pressure-tight manner on the plane connection surface of the bases of the external chamber (22) and the migration chamber(s) (12), for example by glueing.

4. Device according to any one of claims 1 to 3, characterised in that the internal walls of each migration chamber(s) (12) are treated in order to avoid adhesion of cells, such as polynucleate neutrophiles or monocytes, on the said walls.

5. Device according to claim 4, characterised in that the treatment of the said walls is a silicone treatment.

6. A device according to any one of claims 1 to 5, characterised in that the ducts (16, 18 and 20) of each migration chamber (12) traverse the space (24) in which circulates the temperature regulating fluid.

7. Device according to any one of claims 1 to 6, characterised in that the first duct (16) opening close to the top of each migration chamber (12) has a diameter sufficiently large for permitting introduction into each migration chamber (12) apparatuses for controlling the temperature, the pressure and/or the degree of oxygen saturation and for permitting sampling of reagents.

8. Device according to any one of claims 1 to 7, characterised in that the first duct (16) opening close to the top of each migration chamber (12) is equiped with apparatus for regulating the system of pressures in the said chamber.

9. Device according to claim 8, characterised in that the said apparatus for regulating the system of pressures is constituted by a syringe containing the reagent of the said device.

10. Device according to any one of claims 1 to 9, characterised in that the ducts (20) opening near the base of each migration chamber (12) extend radially in a plane parallel to the surface plane of connection of the bases of the external chamber (22) and the migration chamber(s) (12), with a substantially constant angular spacing.

## Ansprüche

1. Zelle zur Untersuchung der Zellwanderung, insbesondere der Wanderung von Leukozyten und Monozyten, mit mindestens einer Migrationskrammer (12), die einen Boden mit einer flachrandigen Öffnung (14), eine erste Leitung (16), die in jede Kammer (12) in Nähe ihres Scheitels einmündet, und eine zweite Leitung (18), die ebenfalls in jede Kammer (12) an deren Seitenwand im wesentlichen auf halber Höhe zwischen ihrem Scheitel und ihrem Boden einmündet, wobei die genannten Leitungen (16, 18) mit einem äußeren Verbindungskreis mit eingebauter Umlaufpumpe verbunden sind, dadurch gekennzeichnet, daß sie weiters eine Mehrzahl von Leitungen (20), die in jede Kammer (12) in unmittelbarer Nähe ihres Bodens einmünden, und eine äußere Kammer (22) aufweist, die die Migrationskammer oder Kammern (12) mit Ausnahme der Bodenöffnung (14) zur Gänze umgibt, wobei die äußeren Kammern (22) und die Migrationskammer(n) (12) dichtend miteinander verbunden sind, insbesondere in Höhe ihres Bodens, und zwischen sich einen Raum (24) abgrenzen, durch den ein Fluid zur Temperaturregelung läuft, das zwischen einer Eingangsleitung (26) und einer Ausgangsleitung (28) der äußeren Kammer (22) zirkuliert, daß die Gesamtheit der Leitungen (20), die in Nähe des Bodens jeder Migrationskammer (12) einmünden, mit der zweiten Leitung (18), die auf halber Höhe jeder Kammer (12) einmündet, so verbunden ist, daß eine ständige Bewegung des in jeder Migrationskammer (12) enthaltenen reaktionsfähigen flüssigen Mediums durch Flüssigkeitsströmung gewährleistet ist, und dadurch gekennzeichnet, daß der Rand der im Boden jeder Migrationskammer (12) angeordneten Öffnung (14) so ausgeführt ist, daß die genannte Zelle entweder unter Einschaltung eines Mikroporenfilters (30) mit einer Porosität zwischen 3 und 5 μm auf der Haut oder gegebenenfalls direkt auf einer Schleimhaut angebracht werden kann.

2. Zelle nach Anspruch 1, dadurch gekennzeichnet, daß die Leitungen (20), die in Nähe des Bodens jeder Migrationskammer (12) einmünden, Kapillarrohre mit im wesentlichen gleichem Durchmesser sind.

3. Zelle nach einem der Ansprüche 1 und 2, dadurch gekennzeichnet, daß der genannte Mikroporenfilter (30) dichtend auf der ebenen Verbindungsfläche der Böden der äußeren Kammern (22) und der Migrationskammer(n) (12) befestigt ist, zum Beispiel durch Kleben.

4. Zelle nach einem der Ansprüche 1 bis 3, dadurch gekennzeichnet, daß die Innenwände jeder Migrationskammer(n) (12) so behandelt werden, daß das Festhängen von Zellen, wie zum Beispiel von polynukleären, neutrophilen Zellen oder von Monozyten, an den genannten Wänden verhindert wird.

5. Zelle nach Anspruch 4, dadurch gekennzeichnet, daß die Behandlung der genannten Wände eine Silikonbehandlung ist.

6. Zelle nach einem der Ansprüche 1 bis 5, dadurch gekennzeichnet, daß die Leitungen (16, 18 und 20) jeder Migrationskammer (12) durch den Raum (24) verlaufen, in dem das Fluid für die Temperaturregelung zirkuliert.

7. Zelle nach einem der Ansprüche 1 bis 6, dadurch gekennzeichnet, daß die nahe dem Scheitel jeder

# 0 012 637

Migrationskammer (12) einmündende erste Leitung (16) einen Durchmesser aufweist, der ausreichend groß ist, daß Einrichtungen zur Überwachung von Temperatur, Druck und/oder Sauerstoffsättigungsgrad eingeführt und Reagenzienentnahmen durchgeführt werden können.

8. Zelle nach einem der Ansprüche 1 bis 7, dadurch gekennzeichnet, daß die nahe dem Scheitel jeder Migrationskammer (12) einmündende erste Leitung (16) eine Einrichtung zur Regelung des Drucksystems im Inneren der genannten Kammer aufweist.

9. Zelle nach Anspruch 8, dadurch gekennzeichnet, daß die genannte Einrichtung zur Regelung des Drucksystems von einer Spritze gebildet wird, die das Reagenz der genannten Zelle enthält.

10. Zelle nach einem der Ansprüche 1 bis 9, dadurch gekennzeichnet, daß die nahe dem Boden jeder Migrationskammer (12) einmündenden Leitungen (20) sich radial, mit einem im wesentlichen konstanten Winkelabstand, in eine Fläche erstrecken, die parallel zur ebenen Verbindungsfläche der Böden der äußeren Kammern (22) und der Migrationskammer(n) (12) verläuft.

7

FIG.1

FIG.2

FIG.3

FIG.4

FIG.5